# EUROPEAN PATENT SPECIFICATION

(11) **EP 1 558 214 B1**
(45) Date of publication and mention of the grant of the patent: **12.09.2012**
(21) Application number: 03810716.5
(22) Date of filing: 07.11.2003
(51) Int. Cl.: A61K 9/107, A61K 31/137, A61K 31/192, A61K 47/10, A61K 47/26

(54) **MICROEMULSION CONCENTRATE FOR ORAL ADMINISTRATION OF WATER-INSOLUBLE ANTI-COLD DRUG AND METHOD FOR PREPARING SAME**
MIKROEMULSIONSKONZENTRATE FÜR DIE ORALEVERABREICHUNG VON WASSERUNLÖSLICHEM ARZNEIMITTEL GEGEN ERKÄLTUNGEN UND HERSTELLUNGSVERFAHREN DAFÜR
CONCENTRE A ADMINISTRATION ORALE DE MICROEMULSION DE MEDICAMENT ANTI-RHUME HYDROSOLUBLE

(30) Priority: 08.11.2002 KR 2002069222
(43) Date of publication of application: 03.08.2005
(73) Proprietor: Hanmi Pharm. Co., Ltd., Kyungki-do 445-910 (KR)
(72) Inventor: WOO, Jong Soo Baekseolmaeul 598-1302, Jeongja-dong, Kyungki-do 440-300 (KR); KIM, Ae Guk, Incheon 404-762 (KR)
(74) Representative: Goddar, Heinz J.
(86) International application number: PCT/KR2003/002388
(87) International publication number: WO 2004/041249

(56) References cited:
- EP-A- 1 249 231
- WO-A-99/29316
- WO-A1-99/39700
- WO-A1-02/058480
- US-A- 4 388 307
- US-A- 5 084 293
- US-B1- 6 190 646

## Description

### FIELD OF THE INVENTION

The present invention relates to a microemulsion concentrate for oral administration of a water-insoluble anti-cold drug, which provides an improved bioavailability of the drug, and a method for preparing same. More particularly, it pertains to a microemulsion concentrate comprising a water-insoluble anti-cold drug, a surfactant and an oil, and a method for the preparation thereof comprising the steps of (a) dissolving the water-insoluble anti-cold drug in a co-surfactant to obtain a homogeneous drug solution; (b) adding the surfactant and the oil in the drug solution to obtain a microemulsion pre-concentrate; and (c) removing the co-surfactant from the pre-concentrate.

### BACKGROUND OF THE INVENTION

As an active ingredient of an anti-cold drug for relieving the symptoms of a cold such as fever, pain, inflammation and cough, various compounds including acetaminophen, ibuprofen, dextromethorphan hydrobromide, noscapine hydrochloride, trimetoquinol hydrochloride, guaifenesin, d-chlorpheniramine maleate, carbetapentane citrate, tipepidine citrate, cloperastine hydrochloride, cloperastine fendizoate, tipepidine hibenzate, d,l-methylephedrine hydrochloride, ephedrine hydrochloride, phenylephedrine hydrochloride, pseudoephedrine hydrochloride, phenylpropanolamine, diphexamide, phenylaminopropanol hydrochloride, oxymetazoline, xylometazoline and the like are currently used.

However, these compounds are practically insoluble in water and, therefore, they exhibit a low absorption rate and bioavailability when orally administered in the form of a solid formulation such as a tablet or a capsule. Further, a syrup formulation containing such compounds is not preferred due to its bulkiness. Accordingly, there have been reported a number of methods to make a soft-capsule containing a high-concentrate of these compounds to improve the absorption rate and bioavailability thereof.

For example, Korean Patent Laid-open, Publication No. 1996-7003576 (Publication Date: 1996. 8. 31) discloses a soft capsule of acetaminophen comprising acetaminophen; polyethyleneglycol, propyleneglycol and water as solvents; an alkali metal acetate as an alkalizer for improving the solubility of the drug; and polyvinylpyrrolidone as a polymer for preventing precipitation. Korean Patent Laid-open Publication No. 2000-31737 (Publication Date: 2000. 6. 5) discloses a soft capsule of acetaminophen comprising acetaminophen; PEG-8-glyceryl caprylate/caprate, diethyleneglycol monoethylether and polyethyleneglycol as solvents; and polyvinylpyrrolidone as a polymer for preventing precipitation. Further, Korean Patent Laid-open Publication No. 1999-11219 (Publication Date: 1999. 2. 18) discloses a soft capsule comprising a drug, polyethyleneglycol, a nonionic surfactant, a stabilizer, glycerin, water and polyvinylpyrrolidone.

On the other hand, Korean Patent Laid-open Publication No. 1997-9793 (Publication Date: 1997. 3. 27) discloses a method for preparing a liquid formulation of ibuprofen comprising dissolving ibuprofen in a mixture of polyoxyethylenesorbitan fatty acid ester, polyglycerin fatty acid ester and water, adding polyvinylpyrrolidone thereto, and heating the resulting mixture to about 80 °C . Korean Patent Laid-open Publication No. 1998-73629 (Publication Date: 1998. 11. 5) discloses a method for preparing a liquid formulation of ibuprofen comprising mixing ibuprofen, polyethyleneglycol, a surfactant and polyvinylpyrrolidone and heating the resulting mixture.

However, the above high-concentrate liquid formulations, which are prepared by simple solubilization of the anti-cold drug would fail to provide a desired absorption rate and bioavailability due to the possible precipitation of the drug upon contact with an aqueous body fluid. Further, the absorbability of the drug may exhibit a significant individual variation.

Accordingly, the inventors have endeavoured to develop a drug delivery system for enhancing the bioavailability of a sparingly water-soluble drug.

### SUMMARY OF THE INVENTION

Accordingly, it is an object of the present invention to provide a method for preparing a microemulsion concentrate for oral administration of a water-insoluble anti-cold drug.

### BRIEF DESCRIPTION OF THE DRAWINGS

The above and other objects and features of the present invention will become apparent from the following description of the invention, when taken in conjunction with the accompanying drawings, in which:
Fig. 1 displays the dissolution rates of the inventive ibuprofen preparation of Example 1 and a commercially available ibuprofen preparation (Brufen^{®} tab.) (Fig. 1 a: artificial gastric juice, Fig. 1b: water).
Fig. 2 displays the particle size distribution of the emulsified microparticles formed from the inventive ibuprofen preparation of Example 1 upon contact with an aqueous solution.
Fig. 3 shows the bioavailabilities of the inventive ibuprofen preparation of Example 1 and a commercially available ibuprofen preparation (Brufen^{®} tab.).

### DETAILED DESCRIPTION OF THE INVENTION

In accordance with the present application, there is provided :
1. A method for preparing a microemulsion concentrate for oral administration of a water-insoluble anti-cold drug comprising (a) dissolving the water-insoluble anti-cold drug in a co-surfactant to obtain a homogeneous drug solution; (b) adding a surfactant and an oil in the drug solution to obtain a microemulsion pre-concentrate; and (c) removing the co-surfactant from the pre-concentrate,
   wherein the water-insoluble anti-cold drug : co-surfactant : surfactant : oil ratio by weight is in the range of 1 : 0.5∼20 : 0.5∼10 : 0.04∼1
2. The method of 1, wherein the water-insoluble anti-cold drug is selected from the group consisting of acetaminophen, ibuprofen, S-ibuprofen, dextromethorphan hydrobromide, noscapine hydrochloride, trimetoquinol hydrochloride, guaifenesin, d-chlorpheniramine maleate, carbetapentane citrate, tipepidine citrate, cloperastine hydrochloride, cloperastine fendizoate, tipepidine hibenzate, d,1-methylephedrine hydrochloride, ephedrine hydrochloride, phenylephedrine hydrochloride, pseudoephedrine hydrochloride, phenylpropanolamine and a mixture thereof.
3. The method of 1, wherein the co-surfactant is an organic solvent having a boiling point lower than 100°C.
4. The method of 3, wherein the co-surfactant is ethanol.
5. the method of 1, wherein the surfactant is selected from the group consisting of polyoxyethylene hydrogenated vegetable oils, polyoxyethylene-polyoxypropylene block copolymer; polyoxyethylene-sorbitan-fatty acid esters, polyoxyethylene fatty acid esters, sodium dioctyl sulfosuccinate or sodium lauryl sulfate, phospholipids, trans-esterification products of natural vegetable oil triglycerides and polyalkylene polyols, mono/di-glycerides, sorbitan fatty acid esters and a mixture thereof.
6. The method of 1, wherein the oil is selected from the group consisting of esters of fatty acids and monovalent alkanols, propyleneglycol mono- or di-fatty acid esters, fatty acid triglycerides, mono/di-glycerides, natural vegetable or animal oils, carbohydrates, tocopherols and a mixture thereof.
7. The method of 1, wherein the co-surfactant is removed in step (C) by heating the pre-concentrate at a temperature ranging from 50 to 100°C.

### (1) Active ingredient

As an active ingredient, any one of known water-insoluble anti-cold drugs can be used. For instance, it may be selected from the group consisting of acetaminophen, ibuprofen, S-ibuprofen, dextromethorphan hydrobromide, noscapine hydrochloride, trimetoquinol hydrochloride, guaifenesin, d-chlorpheniramine maleate, carbetapentane citrate, tipepidine citrate, cloperastine hydrochloride, cloperastine fendizoate, tipepidine hibenzate, d,l-methylephedrine hydrochloride, ephedrine hydrochloride, phenylephedrine hydrochloride, pseudoephedrine hydrochloride, phenylpropanolamine and a mixture thereof.

### (2) Co-surfactant

In the present invention, the co-surfactant serves to dissolve the water-insoluble anti-cold drugs (active ingredient) and facilitates the preparation process by decreasing the viscosity of the reaction mixtures, however, is not contained in the finally obtained microemulsion concentrate. The co-surfactant preferably has a boiling point below 100 °C so that it volatilizes easily. Representative examples thereof include alcohols, acetic acid, lactic acid, glycerin, propyleneglycol, acetone, methylene chloride and the like, wherein alcohols, acetic acid, lactic acid, glycerin and propyleneglycol are more preferable since they are non-toxic to human body, and ethanol is most preferable.

### (3) Surfactant

The surfactant for use in the present invention may be any one of the pharmaceutically acceptable surfactants, which stably emulsifies an oil and hydrophilic ingredients such as the co-surfactant in water. Representative examples of the surfactant include:
① polyoxyethylene glycolated natural or hydrogenated vegetable oils such as polyoxyethylene glycolated natural or hydrogenated castor oil(Cremophor^{®}, BASF; and HCO^{®}, Nikkol),
② polyoxyethylene-sorbitan-fatty acid esters wherein fatty acid is mono- or tri-lauric, palmitic, stearic or oleic acid(Tween^{®}, ICI),
③ polyoxyethylene fatty acid esters such as polyoxyethylene stearic acid ester(Myrj^{®}, ICI),
④ polyoxyethylene-polyoxypropylene block copolymer(Poloxamer^{®}, Pluronic^{®} or Lutrol^{®}, BASF),
⑤ sodium dioctyl sulfosuccinate or sodium lauryl sulfate,
⑥ phospholipids,
⑦ trans-esterification products of natural vegetable oil triglycerides and polyalkylene polyols(Labrafil^{®} M, Gattefosse) and Labrasol,
⑧ mono-, di- or mono/di-glycerides such as caprylic/capric acid mono- and di-glycerides(Imwitor^{®}, Hûls), and
⑨ sorbitan fatty acid esters such as sorbitan monolauryl, sorbitan monopalmityl and sorbitan monostearyl esters(Span^{®}, ICI).

Among the above-mentioned surfactants, polyoxyethylene glycolated hydrogenated vegetable oils, polyoxyethylene-polyoxypropylene block copolymer and polyoxyethylene-sorbitan-fatty acid esters are preferred.

### (4) Oil

The oil may be any one of the pharmaceutically acceptable oils which is compatible with the surfactant and stably emulsified in water to form a stable microemulsion. Representative examples of the oil include:
① fatty acid triglycerides, preferably medium chain fatty acid triglycerides, such as fractionated coconut oil(Miglyol^{®} 812N, Hûls) and triacetin,
② mono-, di- or mono/di-glycerides, preferably mono- or di-glycerides of oleic acid,
③ esters of fatty acids and monovalent alkanols, preferably esters of C₈₋₂₀ fatty acids and C₂₋₃ monovalent alkanols, such as isopropyl myristate, isopropyl palmitate, ethyl linoleate and ethyl oleate,
④ natural vegetable or animal oils such as corn oil, olive oil, soybean oil and fish oil,
⑤ carbohydrates such as squalene and squalane,
⑥ tocopherols such as tocopherol or, tocopherol acetate, tocopherol succinate and polyethyleneglycol-1000-tocopherol succinate(TPGS), and
⑦ propyleneglycol mono- or di-fatty acid esters such as propyleneglycol dicaprylate, propyleneglycol monocaprylate, propyleneglycol dilaurate, propyleneglycol isostearate, propyleneglycol monolaurate and propyleneglycol ricinolate etc.

Among the above-mentioned oils, esters of fatty acids and monovalent alkanols and propyleneglycol fatty acid esters are preferred.

In the preparation of the inventive microemulsion concentrate, the active ingredient, the co-surfactant, the surfactant and the oil may be used in amounts corresponding to a weight ratio in the range of 1 : 0.5∼20 : 0.5∼10 : 0.04∼1, preferably, 1 : 2∼10 : 1∼5 : 0.04∼0.5. In the finally obtained microemulsion concentrate wherein the co-surfactant is removed, the weight ratio of the active ingredient, the surfactant and the oil is in the range of 1 : 0.5∼10 : 0.04∼1, preferably, 1:1∼5 : 0.04∼0.5.

Emulsified drug microparticles contained in the microemulsion concentrate are so stable against the pH change that the change of its emulsified state causing the precipitation of the active ingredient doesn't occur. Upon contact with an aqueous solution, the microemulsion concentrate may easily form a microemulsion containing emulsified drug microparticles having an average particle size ranging from 270 to 500 nm, preferably, from 300 to 400 nm. Accordingly, the microemulsion concentrate is pharmaceutically very useful since it provides a remarkably improved bioavailability of the drug when orally administered, and the drug bioavailability thereof is little influenced by pH change, thereby significantly decreasing the influences of the ingested food and individual absorption difference.

The microemulsion concentrate may be formulated into soft or hard capsule, in accordance with any of the conventional procedures. In addition, the microemulsion concentrate may include pharmaceutically acceptable carriers, excipients or other additives for an oral administration, e.g., stabilizer, dissolution aid and anti-oxidants. Examples of the stabilizer include inorganic or organic acids (salts), preferably, phosphoric acid and anhydrous sodium acetate. The microemulsion concentrate may additionally include diluents such as lactose, dextrose, sucrose, sorbitol, cellulose and glycin; lubricating agents such as silica, talc, stearic acids, magnesium or calcium salts of stearic acid, and polyethyleneglycol; coloring agents; flavoring agents; and sweetening agents.

The pharmaceutical formulation may be sterilized and/or additionally include additives such as preservatives, stabilizers, wetting agents or emulsifying promoters, osmotic agents such as salts and/or buffering agents; and other pharmacologically useful materials.

A typical daily dose of the anti-cold drug can be administered in a single dose or in divided doses.

The following Examples are intended to further illustrate the present invention without limiting its scope.

### Example 1: Preparation of Microemulsion Concentrate and Soft Capsule

A soft capsule was prepared using the following ingredients:

| | Quantity(mg/capsule) |
|---|---|
| Ibuprofen | 200 |
| (Ethanol) | (500) |
| Polyoxyethylene-40-hydrogenated castor oil (Cremophor^{®} RH40; BASF) | 100 |
| Fluronic^{®} L-44NF (BASF) | 200 |
| Tween^{®} 20 (ICI) | 200 |
| Propyleneglycol monocaprylate (NIKKOL) | 50 |

Ibuprofen (active ingredient) was uniformly dissolved in ethanol, and other ingredients were added thereto and dissolved to obtain a microemulsion pre-concentrate. Then, the resulting pre-concentrate was heated at 60 °C for 4 hours to remove ethanol and obtain a highly concentrated microemulsion concentrate wherein ibuprofen was completely dissolved. The microemulsion concentrate was filled into a soft capsule in accordance with the conventional method described in the General Preparation Rule of the Korean Pharmacopoeia.

### Example 2: Preparation of Microemulsion Concentrate and Soft Capsule

A soft capsule was prepared by the procedure of Example 1 using the following ingredients:

| | Quantity(mg/capsule) |
|---|---|
| Ibuprofen | 200 |
| (Ethanol) | (500) |
| Polyoxyethylene-40-hydrogenated castor oil (Cremophor^{®} PH40; BASF) | 350 |
| Fluronic^{®} L-44NF (BASF) | 200 |
| Propyleneglycol monocaprylate (NIKKOL) | 50 |

### Example 3: Preparation of Microemulsion Concentrate and Soft Capsule

A soft capsule was prepared by the procedure of Example 1 using the following ingredients:

| | Quantity(mg/capsule) |
|---|---|
| Ibuprofen | 200 |
| (Ethanol) | (500) |
| Labrasol (GATTEFOSSE) | 100 |
| Tween^{®} 20 (ICI) | 250 |
| Propyleneglycol monocaprylate (NIKKOL) | 70 |

### Example 4: Preparation of Microemulsion Concentrate and Soft Capsule

A soft capsule was prepared by the procedure of Example 1 using the following ingredients:

| | Quantity(mg/capsule) |
|---|---|
| Ibuprofen | 200 |
| (Ethanol) | (500) |
| Polyoxyethylene-40-hydrogenated castor oil | 350 |
| (Cremophor^{®} RH40; BASF) | |
| Tween^{®} 20 (ICI) | 200 |
| Ethyl linolate (NIKKOL) | 50 |

### Example 5: Preparation of Microemulsion Concentrate and Soft Capsule

A soft capsule was prepared by the procedure of Example 1 using the following ingredients:

| | Quantity(mg/capsule) |
|---|---|
| Ibuprofen | 200 |
| (Ethanol) | (500) |
| Labrasol^{®} (GATTEFOSSE) | 100 |
| Fluronic^{®} L-44NF (BASF) | 300 |
| Tween^{®} 20 (ICI) | 100 |
| Propyleneglycol monocaprylate (NIKKOL) | 50 |

### Example 6: Preparation of Microemulsion Concentrate and Soft Capsule

A soft capsule was prepared by the procedure of Example 1 using the following ingredients:

| | Quantity(mg/capsule) |
|---|---|
| Ibuprofen | 200 |
| (Ethanol) | (500) |
| Polyoxyethylene-40-hydrogenated castor oil (Cremophor^{®} PH40; BASF) | 200 |
| Fluronic^{®} L-44NF (BASF) | 150 |
| Tween^{®} 20 (ICI) | 150 |
| Propyleneglycol monocaprylate (NIKKOL) | 50 |

### Example 7: Preparation of Microemulsion Concentrate and Hard Capsule

A hard capsule was prepared by the procedure of Example 1 using the following ingredients:

| | Quantity(mg/capsule) |
|---|---|
| Acetaminophen | 325 |
| (Ethanol) | (1500) |
| Polyethyleneglycol 400 | 300 |
| Tween^{®} 20 (ICI) | 40 |
| Polyvinylpyrrolidone K-30 | 100 |
| Anhydrous acetic acid | 20 |
| Triacetin | 15 |

### Example 8: Preparation of Microemulsion Concentrate and Hard Capsule

A hard capsule was prepared by the procedure of Example 1 using the following ingredients:

| | Quantity (mg/capsule) |
|---|---|
| Acetaminophen | 325 |
| (Ethanol) | (1500) |
| Polyethyleneglycol 400 | 130 |
| Labrasol^{®} (GATTEFOSSE) | 35 |
| Polyvinylpyrrolidone K-30 | 230 |
| Triacetin | 35 |

### Example 9: Preparation of Microemulsion Concentrate and Hard Capsule

A hard capsule was prepared by the procedure of Example 1 using the following ingredients:

| | Quantity(mg/capsule) |
|---|---|
| Acetaminophen | 325 |
| (Ethanol) | (1500) |
| Polyethyleneglycol 400 | 140 |
| Labrasol^{®} (GATTEFOSSE) | 30 |
| Polyvinylpyrrolidone K-30 | 225 |
| Propyleneglycol monocaprylate (NIKKOL) | 30 |

### Example 10: Preparation of Microemulsion Concentrate and Hard Capsule

A hard capsule was prepared by the procedure of Example 1 using the following ingredients:

| | Quantity(mg/capsule) |
|---|---|
| Acetaminophen | 325 |
| (Ethanol) | (1500) |
| Polyethyleneglycol 400 | 150 |
| Fluronic^{®} L-44NF (BASF) | 30 |
| Polyvinylpyrrolidone K-30 | 240 |
| Triacetin | 20 |

### Example 11: Preparation of Microemulsion Concentrate and Soft Capsule

A soft capsule was prepared by the procedure of Example 1 using the following ingredients:

| | Quantity(mg/capsule) |
|---|---|
| d-Chlorpheniramine maleate | 2 |
| Dextromethorphan hydrobromide | 15 |
| Trimetoquinol hydrochloride | 2 |
| Noscapine hydrochloride | 20 |
| Guaifenesin | 75 |
| (Ethanol) | (400) |
| Caprylic/Capric mono and di-glyceride (Capmul MCM) | 180 |
| Cremophor^{®} RH40 (BASF) | 220 |
| Ethyl linolate | 20 |
| Miglyol^{®} 812N (Hûls AM.) | 30 |

### Example 12: Preparation of Microemulsion Concentrate and Soft Capsule

A soft capsule was prepared by the procedure of Example 1 using the following ingredients:

| | Quantity(mg/capsule) |
|---|---|
| d-Chlorpheniramine maleate | 2 |
| Dextromethorphan hydrobromide | 15 |
| Trimetoquinol hydrochloride | 2 |
| Noscapine hydrochloride | 20 |
| Guaifenesin | 75 |
| (Ethanol) | (400) |
| Cremophor^{®} RH40 (BASF) | 250 |
| Labrasol^{®} (GATTEFOSSE) | 150 |
| Propyleneglycol monocaprylate (NIKKOL) | 30 |

### Example 13: Preparation of Microemulsion Concentrate and Soft Capsule

A soft capsule was prepared by the procedure of Example 1 using the following ingredients:

| | Quantity(mg/capsule) |
|---|---|
| d-Chlorpheniramine maleate | 2 |
| Dextromethorphan hydrobromide | 15 |
| Trimetoquinol hydrochloride | 2 |
| Noscapine hydrochloride | 20 |
| Guaifenesin | 75 |
| (Ethanol) | (400) |
| Tween^{®} 20 (ICI) | 250 |
| Labrasol^{®} (GATTEFOSSE) | 150 |
| Propyleneglycol monocaprylate (NIKKOL) | 30 |

### Example 14: Preparation of Microemulsion Concentrate and Soft Capsule

A soft capsule was prepared by the procedure of Example 1 using the following ingredients:

| | Quantity(mg/capsule) |
|---|---|
| d-Chlorpheniramine maleate | 2 |
| Dextromethorphan hydrobromide | 15 |
| Trimetoquinol hydrochloride | 2 |
| Noscapine hydrochloride | 20 |
| Guaifenesin | 75 |
| (Ethanol) | (400) |
| Caprylic/Capric mono and di-glyceride (Capmul^{®} MCM; Abitec) | 180 |
| Cremophor^{®} RH40 (BASF) | 220 |
| Ethyl linolate | 50 |

### Test Example 1: Dissolution Test

The capsule prepared in Example 1 and the commercially available Brufen^{®} tablet (Samil Pharm.) as a comparative preparation were subjected to a dissolution test in accordance with the dissolution test method described in Korea pharmacopoeia (the paddle method). Aliquots of each solution were taken at regular intervals and filtered through a 1 µm membrane filter. The amounts of ibuprofen dissolved therein were determined using the following method:
- Test apparatus: Erweka DT 80
- Test solutions: 900 mℓ each of artificial gastric juice (pH 1.2) and water (pH 6.8)
- Temperature of test solutions: 37 ± 0.5 °C
- Rotation speed: 50±2 rpm
- Sampling time: 5, 10, 15, 30, 45 and 60 min.
- Analysis method: liquid chromatography
   - Column: Inertsil ODS2 (150 mm × 4.6 mm)
   - Mobile phase: acetonitrile : 1% chloroacetate (pH 3.0) (65:35 v/v)
   - Injection volume: 20 µℓ
   - Flow rate: 1.0 mℓ/min.
   - Detector: UV 254 nm

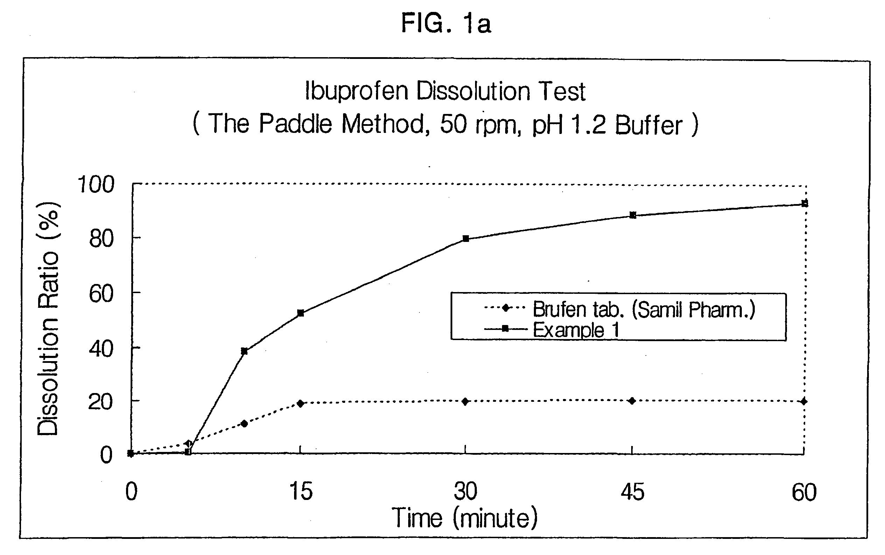

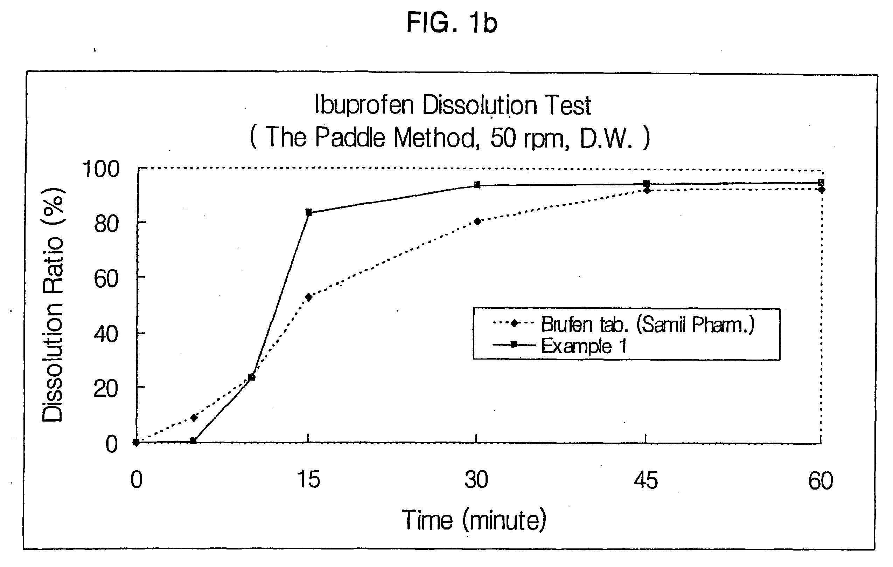

The time-dependent changes in the amount of dissolved ibuprofen, are shown in Figs. 1a and 1b (Fig. 1a: artificial gastric juice, Fig. 1b: water).

As shown in Figs. 1a and 1b, the microemulsion concentrate of Example 1 exhibited higher dissolution rates than the comparative preparation in both test solutions, without the precipitation of the active ingredient due to the pH change.

### Test Example 2: Analysis of the emulsified drug microparticles

In order to examine whether the preparation of Example 1 is spontaneously emulsified to microparticles upon contact with an aqueous solution, a particle size distribution analysis was carried out, as follows.

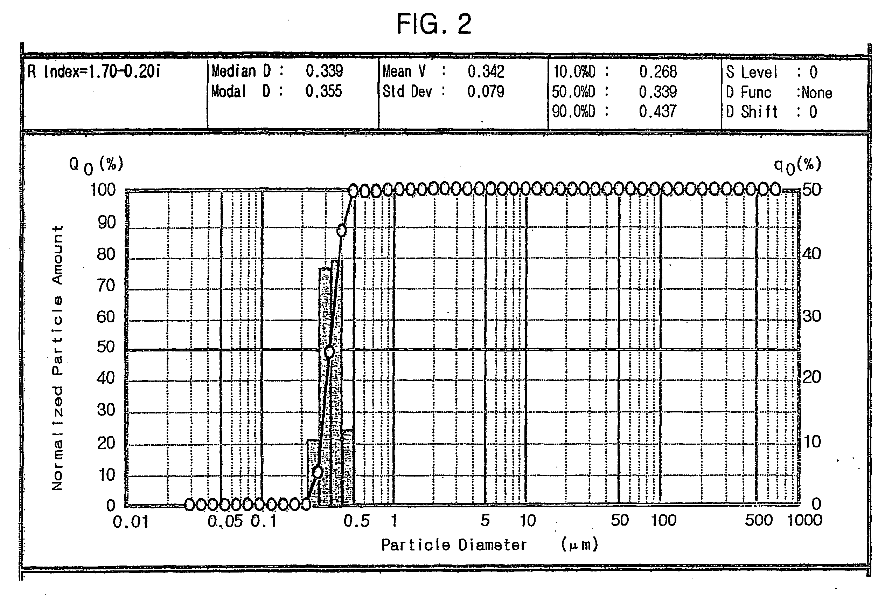

0.1g of the test preparation was diluted with 10 mℓ of distilled water, and then, the particle size distribution was determined with a particle analyzer (Shimadzu, SALD-2002 model, Japan). The result is shown in Fig. 2.

As shown in Fig. 2, the inventive microemulsion concentrate formed an emulsified drug microparticles having an average particle size ranging from about 270 to 500 nm upon contact with an aqueous solution, thereby forming a microemulsion easily.

### Test Example 3: Precipitation Formation Test

In order to examine whether the preparation of Example 1 forms precipitations upon contact with an aqueous solution, each 0.1g of the preparation of Example 1 and the comparative preparation (Brufen^{®} tab.; Samil Pharm.) were diluted to 10 mℓ of distilled water, artificial gastric juice and artificial intestinal juice, respectively, and then, formation of precipitation was observed with eyes immediately.

The artificial gastric juice was prepared by adding 2g of NaCl and 7 mℓ of HCl to 900 mℓ of water to have pH 1.2 and adjusting total volume of the solution to 1 ℓ by adding water thereto. The artificial intestinal juice was prepared by mixing 250 mℓ of 0.2 mol/L KH₂PO₄ and 118 mℓ of 0.2 mol/L NaOH and adjusting pH to 6.8 and adding water thereto to a total volume of 1 ℓ The result of the precipitation formation test is shown in Table 1.

**<Table 1>**

| | Distilled water | Artificial gastric juice | Artificial intestinal juice |
|---|---|---|---|
| Example 1 | - | - | - |

| | | | |
|---|---|---|---|
| (small precipitation: +, large precipitation: ++, no precipitation: -) | | | |

As shown in Table 1, the inventive microemulsion preparation does not form precipitations upon contact with an aqueous solution, therefore the desired absorption rate and bioavailability improvement can be achieved.

### Test Example 4: Absorption Test

In order to investigate the bioavailability of the drug contained in the inventive preparation, in vivo absorption test was carried out as follows by employing the preparation of Example 1 (Experimental preparation) and the commercially available preparation (Brufen^{®}; Samil Pharm.) as a comparative preparation.

Six 14 to 15-week old male Sprague-Dawley rats (weight: 250g) were acclimated for more than 4 days with allowing free access to the feed and water. And then, the rats were put on a fast over 48-hours, while they were allowed to free access to water.

The rats were divided into two groups each consisting of three rats, and the rats were orally administered with the experimental or comparative preparation in an amount corresponding to 20 mg/kg of ibuprofen. Blood samples were taken from the rats before administration, and 15, 30, 45 and 60 min. and 2, 3, 4 and 6 hours after the administration, respectively. 200 µℓ of inner standard solution (10 µg/mℓ propylparaben in methanol) and 200 µℓ of methanol were added to 200 µl of plasma, and the mixture was shaken. The mixture was centrifuged at 3,000 rpm for 10 minutes to obtain a supernant, which was then filtered with a 0.22 µm filter and analyzed by HPLC, as follows.
- Column: Inertsil ODS2 (4.6 mm ×250 mm, 5 µm)
- Mobile phase: 65% acetonitrile solution containing 0.1M sodium acetate
- Injection volume: 20 µℓ
- Flow rate: 1.0 mℓ/min.
- Detector: 222 nm

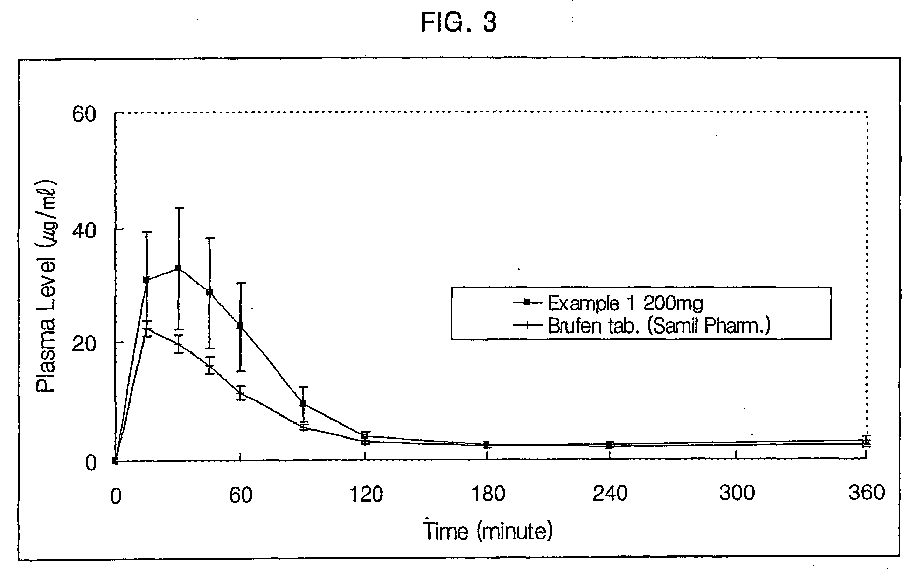

The results is shown in Table 2 and Fig. 3.

**<Table 2>**

| Preparation | AUC (ng·hr/mℓ) | Cₘₐₓ (ng/mℓ) | Tₘₐₓ (hour) |
|---|---|---|---|
| Brufen^{®} tablet (Samil Pharm.) | 2017.7±241.4 | 22.4±2.4 | 15.0±0.0 |
| Example 1 | 2878.4±1300.1 | 34.2±17.6 | 22.5±6.1 |
| AUC: Area under the plasma concentration versus time curve till 24 hours | | | |
| Cₘₐₓ: Maximum blood concentration | | | |
| Tₘₐₓ: Time at the maximum blood concentration | | | |

As shown in Table 2 and Fig. 3, the bioavailability of the inventive preparation of Example 1 was improved than Brufen^{®} tablet.

In considering the fact that, as shown in Figs. 1a and 1b, the inventive microemulsion preparation exhibits a more stable dissolution rate against the pH change than the comparative preparation, can be suggested that the inventive microemulsion concentrate can form stable emulsified drug microparticles upon contact with a body fluid, thereby maintaining the bioavailability constantly without the change of the emulsified state depending on pH, such as precipitation of the drug.

While the invention has been described with respect to the above specific embodiments, it should be recognized that various modifications and changes may be made to the invention by those skilled in the art which also fall within the scope of the invention as defined by the appended claims.

## Claims

1. A method for preparing a microemulsion concentrate for oral administration of a water-insoluble anti-cold drug comprising (a) dissolving the water-insoluble anti-cold drug in a co-surfactant to obtain a homogeneous drug solution; (b) adding a surfactant and an oil in the drug solution to obtain a microemulsion pre-concentrate; and (c) removing the co-surfactant from the pre-concentrate,
wherein the water-insoluble anti-cold drug : co-surfactant : surfactant : oil ratio by weight is in the range of I : 0.5∼20 : 0.5∼10 : 0.04∼1

2. The method of claim 1, wherein the water-insoluble anti-cold drug is selected from the group consisting of acetaminophen, ibuprofen, S-ibuprofen, dextromethorphan hydrobromide, noscapine hydrochloride, trimetoquinol hydrochloride, guaifenesin, d-chlorpheniramine maleate, carbetapentane citrate, tipepidine citrate, cloperastine hydrochloride, cloperastine fendizoate, tipepidine hibenzate, d,l-methylephedrine hydrochloride, ephedrine hydrochloride, phenylephedrine hydrochloride, pseudoephedrine hydrochloride, phenylpropanolamine and a mixture thereof.

3. The method of claim 1, wherein the co-surfactant is an organic solvent having a boiling point lower than 100°C.

4. The method of claim 3, wherein the co-surfactant is ethanol.

5. The method of claim 1, wherein the surfactant is selected from the group consisting of polyoxyethylene hydrogenated vegetable oils, polyoxyethylene-polyoxypropylene block copolymer; polyoxyethylene-sorbitan-fatty acid esters, polyoxyethylene fatty acid esters, sodium dioctyl sulfosuccinate or sodium lauryl sulfate, phospholipids, trans-esterification products of natural vegetable oil triglycerides and polyalkylene polyols, mono/di-glycerides, sorbitan fatty acid esters and a mixture thereof.

6. The method of claim 1, wherein the oil is selected from the group consisting of esters of fatty acids and monovalent alkanols, propyleneglycol mono- or di-fatty acid esters, fatty acid triglycerides, mono/di-glycerides, natural vegetable or animal oils, carbohydrates, tocopherols and a mixture thereof.

7. The method of claim 1, wherein the co-surfactant is removed in step (c) by heating the pre-concentrate at a temperature ranging from 50 to 100°C.

## Patentansprüche

1. Verfahren zur Herstellung eines Mikroemulsionskonzentrats für die orale Verabreichung eines wasserunlöslichen Arzneimittels gegen Erkältungen, welches umfasst, dass (a) das wasserunlösliche Arzneimittel gegen Erkältungen in einem Co-Tensid gelöst wird, um eine homogene Arzneimittellösung zu erhalten; (b) ein Tensid und ein Öl zur Arzneimittellösung zugegeben werden, um ein Mikroemulsionsvorkonzentrat zu erhalten; und (c) das Co-Tensid aus dem Vorkonzentrat entfernt wird,
wobei das Gewichtsverhältnis wasserunlösliches Arzneimittel gegen Erkältungen : Co-Tensid : Tensid : Öl im Bereich von 1 : 0,5-20 : 05-10 : 0,04-1 liegt.

2. Verfahren nach Anspruch 1, wobei das wasserunlösliche Arzneimittel gegen Erkältungen ausgewählt ist aus der Gruppe, bestehend aus Acetaminophen, Ibuprofen, S-Ibuprofen, Dextromethorphan-Hydrobromid, Noscapin-Hydrochlorid, Trimetoquinol-Hydrochlorid, Guaifenesin, d-Chlorpheniramin-Maleat, Carbetapentan-Citrat, Tipepidin-Citrat, Cloperastin-Hydrochlorid, Cloperastin-Fendizoat, Tipepidin-Hibenzat, d,l-Methylephedrin-Hydrochlorid, Ephedrin-Hydrochlorid, Phenylephedrin-Hydrochlorid, Pseudoephedrin-Hydrochlorid, Phenylpropanolamin und einer Mischung davon.

3. Verfahren nach Anspruch 1, wobei das Co-Tensid ein organisches Lösemittel mit einem Siedepunkt von weniger als 100°C ist.

4. Verfahren nach Anspruch 3, wobei das Co-Tensid Ethanol ist.

5. Verfahren nach Anspruch 1, wobei das Tensid ausgewählt ist aus der Gruppe, bestehend aus Polyoxyethylen-hydrierte Pflanzenöle, Polyoxyethylen-Polyoxypropylen-Blockcopolymer, Polyoxyethylensorbitanfettsäureestern, Polyoxyethylenfettsäureestern, Natriumdioctylsulfosuccinat oder Natriumlaurylsulfat, Phospholipiden, Umesterungsprodukten von natürlichen Pflanzenöltriglyceriden und Polyalkylenpolyolen, Mono/Diglyceriden, Sorbitanfettsäureestern und einer Mischung davon.

6. Verfahren nach Anspruch 1, wobei das Öl ausgewählt ist aus der Gruppe, bestehend aus Estern von Fettsäuren und einwertigen Alkanolen, Propylenglykolmono- oder -difettsäureestern, Fettsäuretriglyceriden, Mono/Diglyceriden, natürlichen pflanzlichen oder tierischen Ölen, Kohlehydraten, Tocopherolen und einer Mischung davon.

7. Verfahren nach Anspruch 1, wobei das Co-Tensid in Schritt (c) durch Erhitzen des Vorkonzentrats bei einer Temperatur im Bereich von 50 bis 100°C entfernt wird.

## Revendications

1. Procédé de préparation d'un concentré de microémulsion pour administration par voie orale d'un médicament contre le rhume insoluble dans l'eau comprenant les étapes consistant à (a) dissoudre le médicament contre le rhume dans un co-surfactant afin d'obtenir une solution médicamenteuse homogène ; (b) ajouter un surfactant et une huile dans la solution médicamenteuse afin d'obtenir un pré-concentré de microémulsion ; et (c) retirer le co-surfactant du pré-concentré,
dans lequel le médicament contre le rhume insoluble dans l'eau renferme : co-surfactant : surfactant : rapport d'huile en poids se situant dans la plage de 1 : 0,5 ∼20 : 0,5 ∼ 10 : 0,04 ∼ 1

2. Procédé de la revendication 1, le médicament contre le rhume insoluble dans l'eau étant sélectionné parmi le groupe constitué d'acétaminophène, ibuprofène, S-ibuprofène, bromure hydraté de dextrométhorphane, chlorhydrate de noscapine, chlorhydrate de trimétoquinol, guaifénésine, maléate de d-chlorphéniramine, citrate de carbétapentane, citrate de tipépidine, chlorhydrate de clopérastine, fendizoate de clopérastine, hibenzate de tipépidine, chlorhydrate de d,1-méthyléphédrine, chlorhydrate d'éphédrine, chlorhydrate de phényléphédrine, chlorhydrate de pseudoéphédrine, phénylpropanolamine et leur mélange.

3. Procédé de la revendication 1. le co-surfactant étant un solvant organique ayant un point d'ébullition inférieur à 100°C.

4. Procédé de la revendication 3, le co-surfactant étant un éthanol.

5. Procédé de la revendication 1, le surfactant étant sélectionné parmi le groupe constitué d'huiles végétales hydrogénées de polyoxyéthylène, copolymère bloc de polyoxyéthylène-polyoxypropylène ; esters d'acides gras de polyoxyéthylène sorbitan, esters d'acides gras de polyoxyéthylène, sulfosuccinate de sodium de dioctyle ou sulfate de sodium laurylé, phospholipides, produits de transestérification de triglycérides d'huiles végétales naturelles et polyols de polyalkylène, mono/di-glycérides, esters d'acides gras de sorbitan et leur mélange.

6. Procédé de la revendication 1, l'huile étant sélectionnée parmi le groupe constitué d'esters d'acides gras et d'alkanols monovalents, mono- ou di esters d'acides gras de propylène glycol, triglycérides d'acides gras, mono/di-glycérides, huiles végétales naturelles ou animales, carbohydrates, tocophérols et leur mélange.

7. Procédé de la revendication 1, le co-surfactant étant retiré à l'étape (c) en chauffant le pré-concentré à une température se situant dans la plage de 50 à 100°C.
